(19) Europäisches Patentamt
European Patent Office
Office européen des brevets

(11) Veröffentlichungsnummer : **0 047 452**
**B1**

(12) # EUROPÄISCHE PATENTSCHRIFT

(45) Veröffentlichungstag der Patentschrift : **21.03.84**

(51) Int. Cl.³ : **C 07 D251/34**, C 08 G 18/79

(21) Anmeldenummer : **81106727.1**

(22) Anmeldetag : **28.08.81**

(54) **Verfahren zur Herstellung von Isocyanatgruppen aufweisenden Isocyanuraten die nach diesem Verfahren erhaltenen Verbindungen sowie ihre Verwendung als Isocyanatkomponente in Polyurethanlacken.**

(30) Priorität : **09.09.80 DE 3033860**

(43) Veröffentlichungstag der Anmeldung : **17.03.82 Patentblatt 82/11**

(45) Bekanntmachung des Hinweises auf die Patenterteilung : **21.03.84 Patentblatt 84/12**

(84) Benannte Vertragsstaaten : **AT BE CH DE FR GB IT LI NL SE**

(56) Entgegenhaltungen :
**DE-A- 2 325 826**
**DE-A- 2 644 684**
**DE-A- 2 724 914**
**DE-A- 2 726 749**
**DE-A- 2 901 479**
**GB-A- 952 931**

(73) Patentinhaber : **BAYER AG**
**Konzernverwaltung RP Patentabteilung**
**D-5090 Leverkusen 1 Bayerwerk (DE)**

(72) Erfinder : **Breidenbach, Peter, Dr.**
**Maarweg 33**
**D-5000 Koeln 41 (DE)**
Erfinder : **Bock, Manfred, Dr.**
**Haydnstrasse 18**
**D-5090 Leverkusen 1 (DE)**
Erfinder : **Pedain, Josef, Dr.**
**Haferkamp 6**
**D-5000 Koeln 80 (DE)**
Erfinder : **Mennicken, Gerhard, Dr.**
**Am Wasserturm 16**
**D-5090 Leverkusen 3 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

# Verfahren zur Herstellung von Isocyanatgruppen aufweisenden Isocyanuraten, die nach diesem Verfahren erhaltenen Verbindungen, sowie ihre Verwendung als Isocyanatkomponente in Polyurethanlacken

Die vorliegende Erfindung betrifft ein neues Verfahren zur Herstellung von Isocyanatgruppen aufweisenden Isocyanuraten auf Basis von Gemischen von Hexamethylen-diisocyanat und 1-Isocyanato-3,3,5-trimethyl-5-isocyanato-methyl-cyclohexan (Isophorondiisocyanat, abgekürzt : IPDI), die nach diesem Verfahren erhaltenen Verbindungen, sowie ihre Verwendung als Isocyanatkomponente in Polyurethan lacken.

Die Herstellung von Isocyanatgruppen aufweisenden Isocyanuraten durch katalytische Trimerisierung eines Teils der Isocyanatgruppen von organischen Diisocyanaten ist aus zahlreichen Vorveröffentlichungen bekannt (vgl. z. B. GB-A-809 809 oder GB-A-856 372, DE-C-1 201 992, DE-A-1 644 809, DE-A-1 670 667, DE-A-2 616 415, DE-A-2 616 416, DE-A-2 644 684, DE-A-2 724 914, DE-A-2 726 749, DE-A-2 806 731, US-A-2 801 244, US-A-3 394 111 oder EP-A-10 589).

Die nach diesen Verfahren des Standes der Technik hergestellten, Isocyanatgruppen aufweisende Isocyanurate sind wertvolle Rohstoffe insbesondere zur Herstellung von Zweikomponenten-Polyurethanlacken, in denen sie vorteilhaft als Isocyanatkomponente eingesetzt werden können. Die Isocyanato-Isocyanurate mit aromatisch gebundenen Isocyanuratgruppen eignen sich sehr gut zur Herstellung von Lacken mit hoher Härte und Elastizität, während die Isocyanatoisocyanurate mit aliphatisch oder cycloaliphatisch gebundenen Isocyanatgruppen insbesondere zur Herstellung von wetter- und lichtbeständigen Lacken Einsatz finden. Die in den genannten Vorveröffentlichungen konkret beschriebenen Isocyanato-Isocyanurate mit aliphatisch bzw. cycloaliphatisch gebundenen Isocyanatgruppen sind jedoch noch mit dem Nachteil behaftet, daß sie nicht allen Forderungen der Praxis gleichzeitig gerecht werden. So sind beispielsweise die Isocyanato-Isocyanurate auf Basis von aliphatischen Diisocyanaten wie z. B. Hexamethylendiisocyanat für die an Umfang und Bedeutung immer mehr zunehmenden Anwendungsgebiete, in denen gute Löslichkeit in schwach polaren, physiologisch unbedenklichen Lösungsmitteln (z. B. Benzinfraktionen) gefordert wird, nicht immer geeignet, da zur Herstellung ihrer Lösungen höherpolare Lösungsmittel mitverwendet werden müssen, oder da die Verdünnbarkeit ihrer Lösungen mit den vorgenannten apolaren Lösungsmitteln begrenzt ist.

Zudem sind diese Isocyanato-Isocyanurate in aller Regel flüssig (auch in Form ihrer Umsetzungsprodukte mit Blockierungsmitteln) und aus diesem Grunde für die Anwendung in Pulverlacken nicht geeignet. Überdies zeigen Lacke, die mit diesen Polyisocyanaten hergestellt werden, meist nur geringe Anfangshärten, die ihre rasche Weiterverarbeitung erschweren.

Isocyanato-Isocyanurate, die durch Trimerisierung von cycloaliphatischen Diisocyanaten hergestellt werden, zeigen zwar gute Löslichkeiten in schwach polaren Lösungsmitteln, benötigen aber zur Herstellung von Lösungen mit üblichen Verarbeitungsviskositäten wiederum erhebliche Mengen dieser Löser. Dieses schränkt deren Verwendbarkeit in lösungsmittelarmen Systemen erheblich ein.

Die Produkte besitzen zwar Erweichungspunkte, die deutlich über Raumtemperatur liegen. Sie verleihen jedoch den mit ihnen hergestellten Beschichtungen eine gewisse Sprödigkeit und eingeschränkte Elastizität, die ihre Anwendungen besonders in Lacken, an die auch bei tiefen Temperaturen hohe Forderungen bezüglich ihrer Elastizität gestellt werden, einschränken.

Überraschenderweise wurde jetzt gefunden, daß sich die nachstehend näher beschriebenen, erfindungsgemäßen Isocyanato-Isocyanurate auf Basis von Hexamethylendiisocyanat und IPDI von allen in den genannten Vorveröffentlichungen konkret beschriebenen Isocyanato-Isocyanuraten durch ihr günstiges Eigenschaftsbild vorteilhaft abheben. Die erfindungsgemäßen Verbindungen bzw. Gemische lassen sich zu klaren, hochkonzentrierten und niedrigviskosen Lösungen in schwach polaren Lösungsmitteln lösen, die im Bedarfsfall mit den gleichen Lösungsmitteln beliebig weiterverdünnt werden können, ohne daß Trübungen auftreten. Die mit den erfindungsgemäßen Verbindungen bzw. Gemischen hergestellten Lacke zeichnen sich darüber hinaus durch eine gute Härte und hervorragende Elastizität auch im Bereich tiefer Temperaturen der mit ihnen hergestellten Lacke aus.

Diese Beobachtung ist überraschend, obwohl in einigen der obengenannten Literaturstellen beispielsweise in GB-A-809 809, oder den DE-A-2 644 684, DE-A-2 724 914 oder DE-A-2 726 749 sich nach der Aufzählung lange Listen geeigneter aliphatischer bzw. cycloaliphatischer Diisocyanate auch der lapidare Hinweis findet, daß auch Gemische der genannten Isocyanate eingesetzt werden können. Aus diesem Hinweis allein konnte der Fachmann jedoch keinerlei Anregung entnehmen, dieser Liste ausgerechnet Hexamethylendiisocyanat und IPDI zu entnehmen, um aus diesen beiden Diisocyanaten Mischtrimerisate herzustellen. In der Tat findet sich in den genannten Vorveröffentlichungen keinerlei konkreter Hinweis, diese beiden Diisocyanate auszuwählen.

Die DE-A-2 901 479 beschreibt neben Homotrimerisaten auch Mischtrimerisate aliphatischer Diisocyanate, wobei im Falle der Herstellung von Mischtrimerisaten Isomerengemische von bestimmten aliphatischen Diisocyanaten mit 10 Kohlenstoffatomen im aliphatischen Kohlenwasserstoffrest eingesetzt werden. Die hierbei eingesetzten, sich nur durch Isomerie unterscheidenden Ausgangsdiisocyanate fallen als herstellungsbedingtes Gemisch schon auf der gemeinsamen Aminstufe als Isomerengemisch an. Derartige Isomere unterscheiden sich prinzipiell wenig bezüglich ihrer anwendungstechnischen Eigenschaften. Dies geht auch beispielsweise aus den Ausführungsbeispielen der genannten Vorveröffentlichung hervor, die unabhängig vom gewählten Isomerenverhältnis Verfahrensprodukte beschreiben, die sich bezüglich ihrer anwendungstechnischen Eigenschaften kaum unterscheiden. Der Hinweis auf Seite 15 der Vorveröffentlichung, daß die beschriebenen Polyisocyanate eine bessere Löslichkeit in unpolaren Lösungsmitteln aufweisen bezieht sich, da die Vorveröffentlichung auch die Herstellung von Homotrimerisaten beschreibt, selbstverständlich nicht auf einen, auf das Vorliegen von Mischtrimerisaten zurückzuführenden Vorteil, sondern auf den Umstand, daß gemäß Vorveröffentlichung spezielle $C_{10}$-Alkylen-diisocyanate mit verzweigter Kohlenstoffkette als Ausgangsmaterialien eingesetzt werden, was zu einer besseren Löslichkeit der Trimerisierungsprodukte im Vergleich zu Trimerisierungsprodukten auf Basis von Hexamethylendiisocyanat führt.

Der DE-A-2 901 479 ist nicht der geringste richtungsweisende Hinweis auf das nachstehend näher beschriebene erfindungsgemäße Verfahren zu entnehmen, bei welchem keineswegs Isomerengemische sondern Gemische völlig unterschiedlicher Ausgangsdiisocyanate mischtrimerisiert werden.

Gegenstand der Erfindung ist ein Verfahren zur Herstellung von Isocyanatgruppen aufweisenden Isocyanuraten durch Trimerisierung eines Teils der Isocyanatgruppen eines aus aliphatischen und cycloaliphatischen Diisocyanaten bestehenden Diisocyanatgemischs in Gegenwart von die Trimerisierung von Isocyanatgruppen beschleunigenden Katalysatoren, Abbruch der Trimerisierungsreaktion, sobald 10-60 % der ursprünglich vorliegenden Isocyanatgruppen trimerisiert sind, wobei der eingesetzte Katalysator durch Erhitzen des Reaktionsgemischs auf eine oberhalb der Zersetzungstemperatur des Katalysators liegende Temperatur und/oder durch Zugabe eines Katalysatorengifts, desaktiviert wird und, gegebenenfalls anschließende Entfernung des nicht-umgesetzten Isocyanat-Überschusses durch Dünnschichtdestillation, dadurch gekennzeichnet, daß man ein Gemisch aus Hexamethylendiisocyanat und 1-Isocyanato-3,3,5-trimethyl-5-isocyanatomethyl-cyclohexan im Molverhältnis 4 : 1 bis 1 : 4 einsetzt.

Gegenstand der Erfindung sind auch die nach diesem Verfahren erhaltenen, Isocyanatgruppen aufweisenden Isocyanurate.

Gegenstand der Erfindung ist schließlich auch die Verwendung der nach dem Erfahren erhaltenen, Isocyanatgruppen aufweisenden Isocyanurate, gegebenenfalls in mit Blockierungsmitteln für Isocyanatgruppen blockierter Form, als Isocyanatkomponente in Polyurethanlacken.

Die beim erfindungsgemäßen Verfahren erhaltenen Verbindungen bzw. Gemische von Verbindungen entsprechen der Struktur

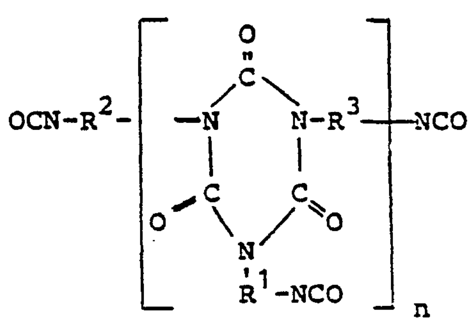

wobei

$R^1$, $R^2$ und $R^3$ für jeweils einen Hexamethylenrest und/oder einen Rest der Formel

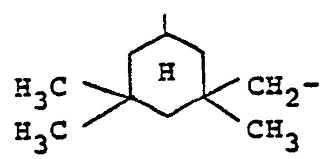

stehen, wobei im Falle des Vorliegens von mit diesen zuletzt genannten Rest verknüpften Isocyanatgruppen diese vorzugsweise direkt mit dem cycloaliphatischen Ring verknüpft sind, und wobei (im statistischen Mittel) mindestens 20 und höchstens 80 %, vorzugsweise mindestens 1/4 und höchstens 2/4 und besonders bevorzugt mindestens 1/3 und höchstens 2/3 der Reste $R^1$, $R^2$ und $R^3$ für einen Hexamethylenrest stehen, und

n für eine ganze oder (im statistischen Mittel) gebrochene Zahl von 1 bis 7, vorzugsweise 1 bis 5, besonders bevorzugt von 1 bis 3 und insbesondere von 1 bis 2 steht.

Wie gaschromatographische und/oder refraktometrische Untersuchungen der nach der erfindungsgemäßen Umsetzung wiedergewonnenen, nicht umgesetzten Diisocyanatgemische zeigten, entspricht das Verhältnis der beiden Reste in den erfindungsgemäßen Verfahrensprodukte überraschenderweise dem Molverhältnis der Diisocyanate, die als Gemisch beim erfindungsgemäßen Verfahren eingesetzt werden. Dementsprechend werden beim erfindungsgemäßen Verfahren die genannten Diisocyanate in einem zwischen 4 : 1 und 1 : 4, vorzugsweise zwischen 3 : 1 und 1 : 3 und besonders bevorzugt zwischen 2 : 1 und 1 : 2 liegenden Molverhältnis eingesetzt.

Die erfindungsgemäßen Verfahrensprodukte stellen in Form ihrer Lösungen in überschüssigen

monomeren Diisocyanaten klare, nahezu farblose und niedrigviskose Flüssigkeiten dar. Nach Entfernung der Diisocyanate durch an sich bekannte Methoden, wie etwa Dünnschichtdestillation, erhält man die Polymerisate als praktisch farblose Harze, deren Gehalt an Ausgangsdiisocyanaten unter 5 Gew.-%, vorzugsweise unter 2 Gew.-% liegt.

Die Konsistenz der Harze hängt vom Molverhältnis der Ausgangskomponenten ab. Im allgemeinen liegt der Schmelzpunkt fester Produkte unter 100 °C und sinkt mit steigendem Anteil an Hexamethylendiisocyanat (HDI). Produkte mit überwiegendem Anteil an HDI sind bei Raumtemperatur meistens flüssig.

Der NCO-Gehalt der erfindungsgemäßen Verfahrensprodukte hängt ebenfalls von deren Zusammensetzung ab und liegt im allgemeinen zwischen 12 und 22 Gew.-%, vorzugsweise zwischen 14 und 21 Gew.-%.

Genauen Aufschluß über die Zusammensetzung der erfindungsgemäßen Verbindungen bzw. Gemische geben gelchromatographische Untersuchungen. Danach stellen im allgemeinen einen Isocyanuratring aufweisende Triisocyanate (n = 1) die Hauptkomponente der monomerenfreien Trimerisate dar. Je nach Umsetzungsgrad bei der Herstellung liegen daneben unterschiedliche Mengen an mehr als einen Isocyanuratring aufweisenden Polyisocyanaten (n = 2-7) vor.

Ausgangsprodukte für das erfindungsgemäße Verfahren sind Hexamethylendiisocyanat (HDI) und IPDI.

Zur Durchführung des erfindungsgemäßen Verfahrens werden die Gemische der Ausgangsdiisocyanate in an sich bekannter Weise mit Hilfe von die Trimerisierung von Isocyanatgruppen beschleunigenden Katalysatoren einer Trimerisierungsreaktion unterworfen.

Als Katalysatoren kommen für das erfindungsgemäße Verfahren alle an sich bekannten Trimerisierungskatalysatoren in Betracht. Als Beispiele für derartige Trimerisierungskatalysatoren seien genannt Phosphine, wie in der DE-A-1 934 763 beschrieben, Alkali- oder Bleisalze gemäß GB-A-809 809, Alkaliphenolate (GB-A-1 391 066, GB-A-1 386 399), Kombinationen aus Alkylenoxid und N,N'-Endoethylenpiperazin (DABCO) (DE-A-2 644 684 und US-A-3 211 703), Aziridin (-derivate) in Kombination mit einem tertiären Amin (DE-A-2 825 826).

Sehr gut geeignet für das erfindungsgemäße Verfahren erweist sich die Verwendung quaternärer Ammonium-hydroxide der allgemeinen Formel

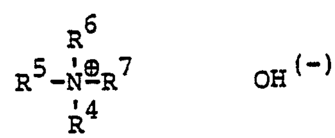

in welcher

$R^4$ für einen Alkylrest mit 1-20, vorzugsweise 4-12 Kohlenstoffatomen, einen araliphatischen Kohlenwasserstoffrest mit 7-10, vorzugsweise 7 Kohlenstoffatomen, oder einen gesättigten cycloaliphatischen Kohlenwasserstoffrest mit 4-10, vorzugsweise 5-6 Kohlenstoffatomen steht, von denen jeder mit Hydroxy- und/oder Hydroxyalkylgruppen mit 1-4 Kohlenstoffatomen substituiert sein kann ;

$R^5$, $R^6$ und $R^7$ für gleiche oder verschiedene Reste stehen und, gegebenenfalls Hydroxyl-substituierte Alkylreste mit 1-20, vorzugsweise 1-4 Kohlenstoffatomen bedeuten, wobei zwei der genannten Reste $R^5$, $R^6$ oder $R^7$ auch zusammen mit dem Stickstoffatom gegebenenfalls zusammen mit einem Sauerstoff- oder einem weiteren Stickstoff-Heteroatom einen heterocyclischen Ring mit 3-5 Kohlenstoffatomen bilden können, oder wobei die Reste $R^5$, $R^6$ und $R^7$ jeweils für Ethylenreste stehen, die zusammen mit dem quaternären Stickstoffatom und einem weiteren tertiären Stickstoffatom ein bicyclisches Triethylen-Diamin (DABCO) — Gerüst bilden können.

Bevorzugte quaternäre Ammoniumhydroxide sind solche der obengenannten Formel, wobei die Reste $R^5$, $R^6$ und $R^7$ die bereits genannte Bedeutung haben, jedoch mit der Maßgabe, daß mindestens einer der genannten Reste mindestens eine aliphatisch gebundene Hydroxylgruppe aufweist, die vorzugsweise in 2-Stellung zum quaternären Stickstoffatom angeordnet ist, wobei der Hydroxyl-substituierte Rest, bzw. die Hydroxylsubstituierten Reste außer den Hydroxyl-Substituenten auch beliebige andere Substituenten, insbesondere $C_1$-$C_4$-Alkoxy-Substituenten, aufweisen können.

Besonders bevorzugte quaternäre Ammoniumhydroxide sind solche der obengenannten Formel, in welchen die Reste $R^4$, $R^5$ und $R^6$ jeweils für Alkylreste der genannten Art und $R^7$ einen Hydroxyethyl-, Hydroxypropyl- oder Hydroxybutylrest bedeuten, in welchem die Hydroxylgruppe vorzugsweise in 2-Stellung zum quaternären Stickstoffatom angeordnet ist.

Beispiele geeigneter quaternärer Ammoniumhydroxide sind Tetramethyl-, Tetraethyl-, Trimethylstearyl-, Dimethyl-ethyl-cyclohexyl-ammoniumhydroxid, N,N,N-Trimethyl-N-(2-hydroxyethyl)-, N,N,N-Trimethyl-N-(2-hydroxypropyl)-, N,N,N-Trimethyl-(2-hydroxybutyl)-ammoniumhydroxid, N,N-Dimethyl-N-dodecyl-N-(2-hydroxyethyl)-ammonium-hydroxid, N-(2-Hydroxyethyl)-N,N-dimethyl-N-(2,2'-dihydroxymethylbutyl)-ammoniumhydroxid, N-Methyl-2-hydroxyethyl-morpholiniumhydroxid, N-Methyl-N-(2-hydroxypropyl)-pyrrolidiniumhydroxid, N-Dodecyl-tris-N-(2-hydroxyethyl)-ammoniumhydroxid, Tetra-(2-hydroxyethyl)-ammoniumhydroxid, sowie die Verbindungen der Formel

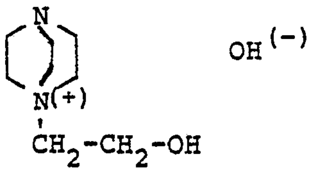

die das Monoaddukt von Ethylenoxid und Wasser an DABCO darstellt.

Die bevorzugt einzusetzenden quaternären Ammonium-hydroxide gestatten eine lösungsmittelfreie Trimerisierung der erfindungsgemäß einzusetzenden Diisocyanatgemische, die in guter Ausbeute unter leicht kontrollierbaren Reaktionsbedingungen (sofortiger Beginn der Trimerisierung bei Katalysatorzugabe) zu kaum gefärbten Produkten führt. Zudem sind die verwendeten Katalysatoren thermolabil, so daß bei Überschreitung gewisser Grenztemperaturen eine selbstständige Desaktivierung eintritt. Dadurch kann die Trimerisierungsreaktion ohne oder mit nur geringen Mengen eines Abstoppers nach dem Stand der Technik beendet werden, was den Vorteil hat, daß im Verfahrensprodukt keine Trübungen auftreten.

Die Trimerisierungskatalysatoren werden im allgemeinen in Mengen von 0,000 1-5, vorzugsweise 0,001-2 Gew.-%, bezogen auf Diisocyanatgemisch, eingesetzt. Bei Verwendung von quaternären Ammoniumhydroxiden als Katalysatoren werden diese im allgemeinen in einer Menge von 0,000 1-2, vorzugsweise 0,001-1 Gew.-%, bezogen auf eingesetztes Diisocyanatgemisch, eingesetzt.

Bei Verwendung von quaternären Ammoniumhydroxiden als Katalysatoren werden diese vorzugsweise in geeigneten Lösungsmitteln gelöst eingesetzt. Als Lösungsmittel eignen sich beispielsweise Toluol, Dimethylformamid, Dimethylsulfoxid oder auch Mischungen davon, die in Mengen von maximal 5 Gew.-%, bezogen auf eingesetztes Isocyanatgemisch, eingesetzt werden, und im Anschluß an die Umsetzung, gegebenenfalls zusammen mit den überschüssigen Diisocyanaten, destillativ entfernt werden.

Erfindungsgemäß bevorzugt sind jedoch Lösungsmittel mit reaktionsfähigen Wasserstoffatomen, speziell Alkohole wie Ethanol, Propanol, Butanole, 2-Ethyl-hexanol, die beim Eintragen in das zu trimerisierende Gemisch der Diisocyanate zu Carbamidsäurederivaten abreagieren können und im Produkt verbleiben.

Die Mitverwendung größerer Mengen an Lösungsmitteln, die im Anschluß an die Umsetzung destillativ entfernt werden können, ist erfindungsgemäß weniger bevorzugt.

Das erfindungsgemäße Verfahren wird bei Temperaturen von 20-120, vorzugsweise 40-100 °C durchgeführt.

Im folgenden wird das erfindungsgemäße Verfahren beispielhaft beschrieben : Eine Mischung aus Hexamethylendiisocyanat und IPDI wird unter Inertgas (dessen Mitverwendung nicht unbedingt erforderlich ist) auf eine Temperatur im Bereich von 20-90 °C, beispielsweise 50 °C gebracht. In die Reaktionslösung trägt man die bevorzugt einzusetzende Lösung des quaternären Ammonium-hydroxid-Katalysators ein, worauf augenblicklich die Trimerisierung beginnt. Die Temperatur steigt dabei auf 60-120 °C an und kann durch geeignete Maßnahmen, bei Reaktionsbeginn bevorzugt durch Kühlung, bei Nachlassen der exothermen Reaktion durch Heizen, bei einem konstanten Wert, beispielsweise 80 °C gehalten werden. Je nach Katalysatormenge und/oder Reaktionstemperatur erreicht der Ansatz in 0,5-5 Stunden den gewünschten NCO-Wert (im allgemeinen bis zu einem Verbrauch von 10-60 %, vorzugsweise 10-40 % der im Anfangsgemisch enthaltenen NCO-Gruppen). Die Trimerisierungsreaktion bricht dann, bevorzugt bei Reaktionstemperaturen oberhalb 75 °C, durch thermische Desaktivierung des Katalysators von selbst ab, oder kann durch Zugabe eines Abstoppers, wie z. B. Säuren oder Säurederivate (Perfluorbutansulfonsäure, Benzoesäure, Benzoylchlorid, Ameisensäure, 2-Ethylhexansäure), besser aber durch kurzzeitiges Erhitzen auf Temperaturen von 80-120 °C unterbrochen werden. Danach wird vorzugsweise die Trimerisatlösung im Hochvakuum, vorzugsweise im Dünnschichtverdampfer, von überschüssigen Monomeren befreit und man erhält als Destillationsrückstand die erfindungsgemäßen Trimerisate.

Ausschlaggebend für die Qualität der Endprodukte (Viskosität und Farbe) ist der schonende Verlauf der Trimerisierung. Die Katalysatormenge wird deshalb so gewählt, daß der NCO-Gehalt nicht zu schnell erreicht wird. Darum Kann es zweckmäßig sein, die Trimerisierung — wie oben beschrieben — nur mit einem Teil der Katalysatormenge zu initiieren, nach Erreichen eines ersten Temperaturmaximums bei dieser Temperatur nachzurühren, bis die Reaktion abklingt, um dann weitere Katalysatorlösung, mit dem Erfolg erneuter Temperaturzunahme, zuzugeben. Ist der gewünschte NCO-Gehalt der Trimerisat-Lösung zu diesem Zeitpunkt noch nicht erreicht, läßt sich nach der gleichen Methode, bei Verwendung thermolabiler Katalysatoren bevorzugt im Bereich von deren spezifischer Zersetzungstemperatur, der Endpunkt einstellen. Selbstverständlich kann dieses Verfahren auch kontinuierlich, etwa in einer Rührkesselkaskade, durchgeführt werden.

Überraschenderweise zeigt sich bei Verwendung der erfindungsgemäß bevorzugt einzusetzenden, Hydroxylgruppen enthaltenden quaternären Ammoniumhydroxide ein Effekt, der — vor allem bei kontinuierlicher Verfahrensweise — eine erhebliche Vereinfachung bedingt : selbst bei Reaktionstemperaturen unterhalb 100 °C, also relativ schonenden Reaktionsbedingungen, zeigen gaschromatographische und/oder refraktometrische Untersuchungen der wiedergewonnenen Monomeren, daß der Anteil der einzelnen Komponenten gegenüber dem Ausgangsgemisch unverändert ist, also dem Molverhältnis der Diisocyanate entsprechend eine stöchiometrische Reaktion stattgefunden hat. Daraus folgt, daß die

wiedergewonnenen Diisocyanate direkt, d. h. ohne aufwendige Analytik und evtl. Korrektur der Zusammensetzung des Diisocyanatgemisches, zur erneuten Verwendung eingesetzt werden können.

Wie gelchromatographische Untersuchungen der erfindungsgemäßen Verfahrensprodukte, d. h. der erfindungsgemäßen Verbindungen bzw. Gemische zeigten, stellen diese ganz überwiegend echte Mischtrimerisate entsprechend der oben angegebenen allgemeinen Formel und nicht etwa Gemische aus HDI- und IPDI-Trimerisaten dar, so daß völlig ausgeschlossen werden kann, daß es sich bei den erfindungsgemäßen Gemischen um Gemische von Verbindungen handelt, für welche jeweils $R^1$, $R^2$ und $R^3$ für gleiche Reste stehen.

Andererseits kann selbstverständlich das Vorliegen derartiger « Homotrimerisate » in untergeordneten Mengen nicht ausgeschlossen werden. Die Angabe, daß die Kohlenwasserstoffreste des HDI bzw. IPDI in den erfindungsgemäßen Verbindungen « im statistischen Mittel » innerhalb der angegebenen Grenzen liegt, bedeutet daher, daß in den erfindungsgemäßen Gemischen eine Mindest- bzw. Höchstmenge an Hexamethylendiisocyanat zusammen mit IPDI in trimerisierter Form vorliegt und zwar vorwiegend in Form echter Mischtrimerisate.

Die erfindungsgemäßen Verbindungen bzw. Gemische stellen wertvolle Ausgangsmaterialien zur Herstellung von Polyurethankunststoffen nach dem Polyadditionsverfahren, insbesondere zur Herstellung von Ein- oder Zwei-Komponenten-Polyurethanlacken, sowie in mit an sich bekannten Blockierungsmitteln blockierter Form wertvolle Ausgangsmaterialien für Zweikomponenten-Polyurethan-Einbrennlacke dar.

Bevorzugte Reaktionspartner für die erfindungsgemäßen, gegebenenfalls in blockierter Form vorliegenden Verbindungen bzw. Gemische bei der Herstellung von Polyurethanlacken sind die in der Polyurethanchemie an sich bekannten Polyhydroxypolyester und -ether, Polyhydroxy-polyacrylate, Polycarbonsäuren und gegebenenfalls niedermolekulare, mehrwertige Alkohole. Auch Polyamine, insbesondere in blockierter Form als Polyketimine oder Oxazolidine sind brauchbare Reaktionspartner für die erfindungsgemäßen Mischpolymerisate.

Die Mengenverhältnisse werden im allgemeinen so gewählt, daß auf eine, gegebenenfalls blockierte, Isocyanatgruppe 0,8-3, vorzugsweise 0,9-1,1 Hydroxy-, Amino- und/oder Carboxylgruppen entfallen.

Zur Beschleunigung der Aushärtung können in bekannter Weise die in der Isocyanatchemie üblichen Katalysatoren verwendet werden, wie z. B. tert. Amine wie Triethylamin, Pyridin, Methylpyridin, Benzyldimethylamin, N,N-Endoethylenpiperazin, N-Methylpiperidin, Pentamethyl-diethylentriamin, N,N-Dimethylaminocyclohexan, N,N'-Dimethylpiperazin oder Metallsalze wie Eisen (III)-chlorid, Zinkchlorid, Zink-2-ethylcaproat, Zinn(II)-ethylcaproat, Dibutylzinn (IV)-dilaurat, Molybdänglykolat.

Bei Verwendung der erfindungsgemäßen Verbindungen bzw. Gemische in Einbrennlacken werden deren NCO-Gruppen ganz oder teilweise in bekannter Weise blockiert. Hierzu wird das Polyisocyanat mit einem geeigneten Blockierungsmittel, vorzugsweise bei erhöhter Temperatur, gegebenenfalls in Gegenwart eines geeigneten Katalysators (s. o.) umgesetzt.

Geeignete Blockierungsmittel sind beispielsweise Monophenole (Phenol, Kresole), tertiäre Alkohole (t.-Butanol, Dimethylphenylcarbonol), leicht Enole bildende Verbindungen (Acetessigester, Malonsäurederivate), sekundäre, aromatische Amine (N-Methylanilin, N-Phenyl-xylidin), Imide (Succinimid), Lactame (ε-Caprolactam, δ-Valerolactam), Oxime (Butanonoxim, Cyclohexanonoxim), Mercaptane (Methylmercaptan, Ethylmercaptan) oder Triazole (1H-1,2,4-triazol).

Zur Herstellung der Lackbindemittel werden beispielsweise gegebenenfalls blockiertes Polyisocyanat, polyfunktionelle Reaktionspartner, Katalysator und gegebenenfalls die üblichen Zusätze, wie z. B. Pigmente, Füll- und Farbstoffe und Verlaufmittel miteinander auf einem üblichen Mischaggregat, wie z. B. auf einer Sandmühle entweder mit oder ohne Lösungs- und Verdünnungsmittel gut vermischt und homogenisiert.

Die Anstrich- und Überzugsmittel können in Lösung oder aus der Schmelze oder in fester Form nach den üblichen Methoden wie z. B. Streichen, Rollen, Gießen, Spritzen, dem Wirbelsinterverfahren oder dem elektrostatischen Pulversprühverfahren auf den zu beschichtenden Gegenstand aufgebracht werden.

Bei Verwendung blockierter Polyisocyanate in Pulverlacken, die bevorzugt nach dem elektrostatischen Pulversprühverfahren aufgebracht werden, war es bisher nicht möglich, ausschließlich durch Isocyanuratgruppenbildung modifiziertes HDI als Pulverlackhärter einzusetzen : bei Blockierung reiner HDI-Trimerisate mit ε-Caprolactam in stöchiometrischen Mengen erhält man unabhängig vom Trimerisierungsgrad und unabhängig davon, ob überschüssige Monomere zuvor in einem zusätzlichen Arbeitsgang, etwa durch Dünnschichtdestillation, entfernt werden, bei Raumtemperatur flüssige oder klebrige Produkte, die aufgrund ihres niedrigen Erweichungspunktes als Pulverlackhärter nicht geeignet sind (Vergleichsbeispiele 13, 14). In Form von blockierten Mischtrimerisaten mit cycloaliphatischen Diisocyanaten läßt sich jedoch HDI, über dessen Anteil im Mischtrimerisat z. B. die Elastizität der ausgehärteten Lacke gezielt zu beeinflussen ist, ohne weiteres als Komponente in Pulverlackhärtern verwenden : mit ε-Caprolactam blockierte HDI/IPDI-Mischtrimerisate z. B. sind aufgrund ihres hohen Schmelzpunktes gut zu zerkleinern und bleiben über Wochen rieselfähig (Beispiele 10-12). Ein vergleichsweise mit ε-Caprolactam blockiertes Trimerisatgemisch aus HDI-Trimerisat und IPDI-Trimerisat (Beispiel 15) zeigt im Gegensatz zu einem Mischtrimerisat gleicher Brutto-Zusammensetzung einen deutlich geringeren Schmelzbereich. Das gemahlene Produkt verklebt bei Lagerung bei 40 °C vollständig.

Die monomerenarmen Mischtrimerisate der erfindungsgemäßen Art besitzen eine sehr gute Löslichkeit und Verdünnbarkeit in für Lackanwendungen üblichen Lösungsmitteln wie Ethylglykolacetat, Ethylglykolacetat/Xylol 1 : 1, Essigsäureethylester, -butylester, sowie generell in Aromaten und benzinreichen Gemischen der vorgenannten Lösungsmittel. Selbst hochkonzentrierte Lösungen zeigen dabei niedrige Viskositäten. Ein wesentlicher Vorteil des erfindungsgemäßen Verfahrens ist auch darin zu sehen, daß die Eigenschaften der Verfahrensprodukte auf einfache Weise durch geeignete Wahl des Molverhältnisses der Ausgangsdiisocyanate dem gewünschen Anwendungszweck optimal angepaßt werden können. Der wesentliche Vorteil der erfindungsgemäßen Verbindungen bzw. Gemische ist darin zu sehen, daß in ihnen weitgehend die Vorteile der rein aliphatischen Trimerisate (hohe Elastizität und Schlagfestigkeit der mit ihnen hergestellten Lacke) mit den Vorteilen der bekannten IPDI-Trimerisate (gute Löslichkeit im apolaren Lösungsmitteln bzw. Lösungsmittelgemischen, gute Härte der Lackfilme) nicht jedoch die obengenannten Nachteile kombiniert sind.

Die folgenden Beispiele dienen der weiteren Erläuterung der Erfindung. Alle Prozentangaben beziehen sich auf Gewichtsprozente.

In den nachfolgenden Beispielen werden folgende Katalysatorlösungen eingesetzt :

Katalysator A : N-Dodecyl-N,N-dimethyl-N-(2-hydroxy-ethyl)-ammoniumhydroxid, hergestellt durch Ethoxylierung von N-Dodecyl-N,N-dimethylamin und verdünnt auf eine ca. 5 %ige Lösung mit 2-Ethylhexanol/Ethanol 8 : 1

Katalysator B : N-(2-hydroxyethyl)-N,N-dimethyl-N-(2,2-dihydroxymethyl-butyl)-ammoniumhydroxid, verdünnt auf eine ca. 10 %ige Lösung mit 2-Ethylhexanol

Katalysator C : N,N,N-Trimethyl-N-(2-hydroxyethyl)-ammoniumhydroxid
I verdünnt auf eine ca. 2 %ige Lösung in 2-Ethylhexanol/Ethanol 8 : 1
II verdünnt auf eine ca. 2 %ige Lösung in Dimethylformamid/Ethanol 8 : 1
III verdünnt auf eine ca. 6 %ige Lösung in Dimethylformamid/Ethanol 4 : 1

Katalysator D : N-Trimethyl-N-(2-hydroxypropyl)-ammonium-hydroxid
I verdünnt auf eine ca. 3 %ige Lösung in 2-Ethylhexanol/Ethanol 8 : 1
II verdünnt auf eine ca. 6 %ige Lösung in Ethylhexanol/n-Propanol 8 : 1

## Beispiel 1

Ein Gemisch aus 4 704 g (28 Mol) Hexamethylendiisocyanat und 1 554 g (7 Mol) Isophorondiisocyanat wird bei 65 °C mit 100 ml Katalysator A versetzt. Die Reaktion verläuft sofort exotherm und wird durch Kühlung 10 Minuten bei 80 °C gehalten. Nach Entfernung der Kühlung steigt die Temperatur auf 96 °C an. Nach weiteren 15 Minuten sinkt die Temperatur und wird durch Heizen bei 80 °C gehalten. Nach insgesamt 1 Stunde ist der Katalysator desaktiviert. Der NCO-Gehalt des Gemisches ist dann konstant und beträgt 39,9 %. Das durch Dünnschichtdestillation von monomeren Diisocyanaten fast vollständig befreite Produkt (Restgehalt an HDI : 0,25 %, Restgehalt an IPDI : 0,37 %) hat eine geringe gelbliche Eigenfarbe, einen NCO-Gehalt von 19,8 % und eine Viskosität von 5 320 mPa.s (23 °C).

## Beispiel 2

3 360 g (20 Mol) HDI und 2 220 g (10 Mol) IPDI werden bei 65 °C mit 100 ml Katalysatorlösung A versetzt, wobei die Temperatur langsam auf 82 °C ansteigt. Nach Abklingen der exothermen Reaktion beträgt der NCO-Gehalt des Gemisches 38,5 %. Erneute Zugabe von 30 ml Katalysatorlösung bei 80 °C bewirkt einen Temperaturanstieg auf 98 °C. Nach insgesamt 140 Minuten Reaktionszeit zeigt der Katalysator keine Wirksamkeit mehr, der NCO-Gehalt ist auf 33,1 % gesunken. Das Isocyanatgemisch wird durch Dünnschichtdestillation von Monomeren befreit und 90 % in Ethylglykolacetat/Xylol 1 : 1 gelöst. Die praktisch farblose Lösung besitzt einen NCO-Gehalt von 16,6 % und eine Viskosität von 2 780 mPa.s (23 °C).

## Beispiel 3

444 g (2 Mol) IPDI und 672 g (4 Mol) HDI werden bei 80 °C mit 20 ml Katalysatorlösung B versetzt. Die Reaktion verläuft sofort exotherm und wird durch gelegentliche Kühlung bei 90 °C gehalten. Nach 30 Minuten wird die Reaktion durch Zugabe von 1 ml Abstopperlösung (bestehend aus 1 ml Perfluorbutansulfonsäure in 2 ml Dimethylformamid) unterbrochen. Das Gemisch besitzt dann einen NCO-Gehalt von 33,6 %. Durch Dünnschichtdestillation erhält man ein schwach gelbes Harz, das als praktisch farblose Lösung (90 %) in Ethylglykolacetat/Xylol (1 : 1) einen NCO-Gehalt von 16,5 % besitzt.

## Beispiel 4

1 776 g (8 Mol) IPDI und 2 688 g (16 Mol) HDI werden bei 60 °C mit 120 ml Katalysatorlösung C I versetzt. Die Trimerisierung verläuft sofort exotherm, die Reaktionsmischung hat nach ca. 10 Minuten 75 °C erreicht. Nach weiteren 15 Minuten beträgt die Temperatur 73 °C. Die Reaktion wird bei einem

NCO-Gehalt von 35,7 % durch Zugabe einer Lösung von 2 ml Perfluorbutansulfonsäure in 4 ml Dimethylformamid unterbrochen. Durch Dünnschichtdestillation gewinnt man das Mischtrimerisat in Form eines nahezu farblosen Harzes mit einem Monomerengehalt von 0,11 % HDI und 0,13 % IPDI. Als 90 % Lösung in Ethylglykolacetat/Xylol 1 : 1 besitzt das Harz einen NCO-Gehalt von 17,2 % und eine Viskosität von 1 350 mPa.s (23 °C).

Beispiel 5

Eine Mischung aus 2 520 g (15 Mol) HDI und 3 330 g (15 Mol) Isophorondiisocyanat wird bei 70 °C einmal mit 100, dann mit 40 ml Katalysatorlösung A versetzt. Die Temperatur steigt dabei auf maximal 103 °C an. Nach 140 Minuten Reaktionszeit beträgt der NCO-Gehalt der Lösung 30,8 %. Das durch Dünnschichtdestillation gewonnene Harz wird 80 % in Ethylglykolacetat/Xylol 1 : 1 gelöst. Die nahezu farblose Lösung besitzt einen NCO-Gehalt von 13,8 % und eine Viskosität von 1 440 mPa.s (23 °C). Der Gehalt an freiem HDI beträgt 0,17 %, an freiem IPDI 0,27 % (beide Werte bezogen auf Lösung).

Beispiel 6

1 344 g (8 Mol) HDI und 3 552 g (16 Mol) IPDI werden bei 65 °C vorgelegt. 140 ml Katalysatorlösung A werden portionsweise so zugegeben, daß die Temperatur der Lösung zu keinem Zeitpunkt 85 °C überschreitet. Danach wird die Reaktionsmischung auf 90 °C aufgeheizt und 20 Minuten bei dieser Temperatur belassen. Der NCO-Gehalt der Lösung beträgt 31,5 %. Das Harz wird durch Dünnschichtdestillation von Diisocyanaten befreit und in Ethylglykolacetat/Xylol 1 : 1 gelöst (80 %).

NCO-Gehalt : 13,0 %

$n^{23}$ : 930 mPa.s

Beispiel 7

3 552 g (16 Mol) IPDI und 1 344 g (8 Mol) HDI werden bei 60 °C mit 120 ml Katalysatorlösung C II versetzt. Die Temperatur des Gemisches steigt innerhalb von 15 Minuten auf 74 °C, um dann langsam wieder abzufallen. Nach insgesamt 40 Minuten wird die Reaktion durch Zugabe einer Lösung von 2 ml Perfluorbutansulfonsäure in 4 ml Dimethylformamid abgestoppt und das Rohprodukt einer Dünnschichtdestillation unterworfen. Man erhält ein nahezu farbloses Harz mit einem NCO-Gehalt von 13,8 % und einer Viskosität von 1 040 mPa.s (23 °C).

Beispiel 8

888 g (4 Mol) IPDI und 336 g (2 Mol) HDI werden bei 70 °C mit 40 ml Katalysatorlösung C III versetzt. Die Temperatur des Reaktionsgemisches steigt langsam an und wird anfangs durch gelegentliche Kühlung, dann durch Heizen bei 80 °C gehalten. Nach 50 Minuten wird die Reaktion durch Zugabe einer Lösung von 0,5 ml Perfluorbutansulfonsäure in 1 ml Dimethylformamid abgestoppt, der NCO-Gehalt beträgt dann 33,2 %. Das durch Dünnschichtdestillation isolierte Produkt wird in Ethylglykolacetat/Xylol 1 : 1 gelöst (80 %). Das praktisch farblose Produkt besitzt einen NCO-Gehalt von 13,5 %, eine Viskosität von 620 mPa.s (23 °C), der Gehalt an HDI beträgt 0,16 %, der Gehalt an IPDI 0,32 %.

Beispiel 9

Zu einem Gemisch von 840 g (5 Mol) HDI und 4 440 g (20 Mol) IPDI werden bei 70 °C 130 ml Katalysatorlösung A in zwei Portionen zugegeben. Nach der sofort einsetzenden exothermen Reaktion wird die Temperatur anfangs durch Kühlung, nach Abklingen der exothermen Reaktion durch Heizen bei 80 °C gehalten. Nach 2 Stunden Reaktionszeit beträgt der NCO-Gehalt des Gemisches 30,8 %. Durch 15 Minuten Erhitzen auf 90 °C werden Reste noch aktiven Katalysators zerstört, der NCO-Gehalt beträgt dann 30,2 %. Das durch Dünnschichtdestillation gewonnene monomerenarme Produkt besitzt als 80 %ige Lösung in Ethylglykolacetat/Xylol 1 : 1 einen NCO-Gehalt von 16,6 % und eine Viskosität von 3 780 mPa.s (23 °C).

Beispiel 10

336 g (2 Mol) HDI und 444 g (2 Mol) IPDI werden bei 80 °C mit insgesamt 40 ml Katalysatorlösung D I bis zu einem NCO-Gehalt von 16,8 % trimerisiert. Das Trimerisat wird bei 90-140 °C mit 352 g ε-Caprolactam umgesetzt. Das verkappte Trimerisat schmilzt bei 75-81 °C und hat einen blockierten NCO-Gehalt von 11,6 %. Das gemahlene Produkt bleibt bei Lagerung bei 40 °C nach 3 Wochen noch rieselfähig.

Beispiel 11

168 g (1 Mol) HDI und 444 g (2 Mol) IPDI werden bei 60 °C mit 25 ml Katalysatorlösung D II versetzt.

Die Reaktion startet sofort exotherm, und wird durch gelegentliches Kühlen bei 80 °C gehalten. Nach Beendigung der exothermen Reaktion werden erneut 9 ml Katalysatorlösung zugegeben und das Gemisch — anfangs durch Kühlung, später durch Heizen — bei 80 °C gehalten, bis der NCO-Gehalt konstant ist. Er beträgt dann 20,0 %.

Das Gemisch wird dann auf 100 °C erhitzt. Man läßt 330 g geschmolzenes ε-Caprolactam unter Rühren so zugetropft, daß die Temperatur des Gemisches allmählich bis 140 °C steigt. Nach vollständiger Reaktion läßt man auf Raumtemperatur abkühlen. Man erhält einen spröden, gut zu zerkleinernden Feststoff, der bei 92-96 °C schmilzt. Der NCO-Gehalt beträgt 0 %, der Gehalt an blockierten Isocyanat-Gruppen (berechnet als NCO) 13 %.

Beispiel 12

500 g (2,98 Mol) Hexamethylendiisocyanat und 500 g (2,25 Mol) Isophorondiisocyanat werden bei 60 °C mit 40 ml Katalysatorlösung D I versetzt. Die Reaktion startet sofort exotherm, der Ansatz erreicht eine Temperatur von 86 °C. Man läßt die Temperatur auf 80 °C absinken und hält diese Temperatur durch Heizen. Nach insgesamt 2 Stunden liegt der NCO-Gehalt bei 31,5 %. Nach erneuter Katalysatorzugabe (25 ml) erreicht der Ansatz in weiteren 7 Stunden bei 80 °C einen konstanten NCO-Gehalt von 16,2 %. Der Gehalt an freiem HDI beträgt 6,3 %, der Gehalt an freiem IPDI ca. 6,8 %.

Die Blockierung mit 436 g ε-Caprolactan bei 100-140 °C ergibt einen spröden Feststoff mit einem Schmelzpunkt von 72-78 °C. Der Gehalt an blockierten Isocyanat-Gruppen beträgt 11,3 %. Der gemahlene Feststoff bleibt bei Lagerung bei 40 °C über Wochen rieselfähig.

Beispiel 13 (Vergleichsbeispiel)

1 008 g (6 Mol) HDI werden bei 80 °C mit insgesamt 13 ml Katalysatorlösung D II bis zu einem NCO-Gehalt von 25,5 % trimerisiert und bei 100-130 °C mit 692 g ε-Caprolactam blockiert. Das abgekühlte Produkt ist klebrig und kann nicht durch Mahlen zerkleinert werden.

Beispiel 14 (Vergleichsbeispiel)

504 g (3 Mol) HDI werden bei 80 °C mit 9 ml Katalysatorlösung D II bis zu einem NCO-Gehalt von 30,0 % trimerisiert und durch Dünnschichtdestillation von Monomeren befreit (Restmonomerengehalt : 0,35 %). Der NCO-Gehalt beträgt dann 19,5 %.

Das Produkt wird bei 100-130 °C mit der stöchiometrischen Menge ε-Caprolactam blockiert. Man erhält ein bei Raumtemperatur klebriges Produkt, das nicht als Pulverlackvernetzer verwendet werden kann.

Beispiel 15 (Vergleichsbeispiel)

93,7 g monomerenfreies HDI-Trimerisat, 93,2 g monomerenfreies IPDI-Trimerisat, 6,3 g HDI und 6,8 g IPDI (entsprechend der Zusammensetzung des in Beispiel 12 erhaltenen Trimerisats) werden bei 100 °C homogenisiert. Der NCO-Gehalt des Gemisches beträgt 20,2 %. Nach Blockierung mit 109 g ε-Caprolactam bei 100-140 °C erhält man einen Feststoff, der bei 58-65 °C schmilzt. Das gemahlene Produkt verklebt bei Lagerung bei 40 °C vollständig.

Beispiel 16 (Vergleichsbeispiel)

Dieses Vergleichsbeispiel zeigt, in wie weit Elastizität, Schlagfestigkeit und Härte von Lacken bei gleichbleibender Polyolkomponente durch Anwendung der erfindungsgemäßen Mischtrimerisate verändert werden können. Dazu wurden aus folgenden Ausgangskomponenten die nachstehend aufgeführten Mischungen (Angaben in Gewichtsteilen, NCO : OH = 1 : 1) hergestellt :

1. Mischtrimerisat HDI/IPDI (Molverhältnis 2 : 1) nach Beispiel 4
2. Mischtrimerisat HDI/IPDI (Molverhältnis 1 : 2) nach Beispiel 8
3. IPDI-Trimerisat, 70 % in Ethylglykolacetat/Xylol 1 : 1, NCO-Gehalt : 11,5 %, Gehalt an freiem IPDI : < 0,7 %

Löser : Gemisch aus gleichen Gewichtsteilen Ethylglykolacetat, Xylol, Butylacetat

Hydroxylkomponente : Polyester aus Adipinsäure, Hexandiol und Diphenylcarbonat, OH-Gehalt : 6 Gew.-%

Katalysator : DABCO, 10 % gelöst in Ethylglykolacetat (0,5 % Katalysator, bezogen auf Festharz der Abmischung).

| Abmischung | A | B | C |
|---|---|---|---|
| Zusammensetzung | | | |
| 1 | 86 | - | - |
| 2 | - | 107 | - |
| 3 | - | - | 129 |
| Löser | 108 | 113 | 88 |
| Polyester | 100 | 100 | 100 |
| Katalysator | 8,8 | 9,3 | 9,5 |

An daraus hergestellten (ca. 40 µm Trockenfilm, Spritzapplikation, Aushärtung 14 Tage bei 23 °C) wurde nach deren vollständiger Aushärtung bestimmt:

a) Pendelhärte (nach König ; DIN 53 157) in sec
b) Elastizität (DIN 53 156) in mm
c) Schlagfestigkeit (ASTM D 779-69) in inch x pound

Folgende Werte wurden bestimmt:

| Film | A | B | C |
|---|---|---|---|
| a | 74 | 28 | 174 |
| b | 9,5 | 9,2 | 6,9 |
| c | 80 | 80 | 10 |

Die Ergebnisse zeigen, daß Filme aus den erfindungsgemäßen Mischtrimerisaten eine deutlich verbesserte Elastizität und Schlagfestigkeit besitzen. Weiterhin sieht man, daß allein durch Wahl der Zusammensetzung der erfindungsgemäßen Polyisocyanate bei sonst gleichbleibend guten, bzw. verbesserten Eigenschaften die Härte daraus hergestellter Filme in weiten Grenzen variiert und dem jeweiligen Anwendungszweck optimal angepaßt werden kann.

## Beispiel 17

Dieses Beispiel zeigt, wie die Eigenschaften eines Alkydharzlackes durch Verwendung eines erfindungsgemäßen Polyisocyanats verbessert werden können.

Dazu wird ein handelsübliches Alkydharz auf Basis von Sojafettsäure (Öllänge ca. 48 % berrechnet auf Triglycerid) 50 % in Benzin gelöst und mit Titandioxid pigmentiert (65 % bezogen auf Bindemittel).

Die Verbesserung der Filmeigenschaften erfolgte durch Zugabe eines Mischtrimerisats nach Beispiel 5, das mit Benzin auf eine 40 % Lösung verdünnt war (8 % Polyisocyanat, bezogen auf festes Alkydharz). Danach wurde mit Benzin auf Verarbeitungsviskosität verdünnt. Die Eigenschaften der Abmischungen, bzw. daraus hergestellter Filme gibt die folgende Tabelle wieder:

(Siehe Tabelle Seite 11 f.)

| | Alkydharz-Lack ohne | mit Polyisocyanatzusatz |
|---|---|---|
| Verarbeitungszeit[1)] | unbegrenzt | 1 Arbeitstag |
| Pendelhärte nach 16 Stunden[2)] | 15 sec | 20-30 sec |
| Abklebbarkeit nach 16 Stunden[3)] | schlecht | sehr gut |
| Nagelhärte nach 16 Stunden | schlecht | gut |
| Benzinfestigkeit | schlecht | mittel |
| Überspritzbarkeit[4)] | schlecht | gut |
| Pendelhärte nach 30 Tagen[2)] | ca. 70 sec | ca. 100 sec |
| Glanz | sehr gut | sehr gut |

1) Auslaufzeit nach DIN 53 211 im DIN-Becher 4 mm, Anstieg von 20 sec auf 35 sec

2) Aushärtung bei 23 °C, Bestimmung der Dämpfungsdauer nach König (DIN 53 157).

3) Prüfung mit Tesakreppband 321, angedrückt mit ca. 500 g, nach 15 Minuten entfernt, Abdruck 30 Minuten später bewertet.

4) Filme nach 16 Stunden angeschliffen und mit demselben Lack überspritzt.

Beispiel 18 (Vergleichsbeispiel)

Analog der in Beispiel 17 beschriebenen Verfahrensweise wird der Alkydharzlack mit einem Polyisocyanat versetzt. Anstelle des HDI/IPDI-Mischtrimerisats (Molverhältnis 1 : 1, entsprechend einem Gewichtsverhältnis von 43 : 57) wird jedoch hier ein Trimerisatgemisch aus 43 Teilen eines reinen HDI-Trimerisats und 57 Teilen eines reinen IPDI-Trimerisats verwendet. Aufgrund der weniger guten Verdünnbarkeit der Polyisocyanat-Mischung mit Benzin im Vergleich zu der des Mischtrimerisats wird bereits eine trübe Lösung erhalten, wenn man auf den gleichen Festkörpergehalt wie in Beispiel 28 verdünnt. Die erhaltenen Filme nach Trocknung bei Raumtemperatur, bzw. bei 60 °C zeigen eine starke Beeinträchtigung des Glanzgrades.

Beispiel 19

Die folgende Tabelle beschreibt den Unterschied im Harzcharakter von « reinen » Trimerisaten, Trimerisatgemischen und einem erfindungsgemäßen Mischtrimerisat :

| Polyisocyanat | Harzcharakter bei 23 °C |
|---|---|
| HDI-Trimerisat | flüssig |
| HDI/IPDI-Mischtrimerisat Gew.-verh. 43 : 57 nach Beispiel 5 | zähflüssig |
| HDI/IPDI-Trimerisatgemisch Gew.-verh. 43 : 57 | fest, weich |
| IPDI-Trimerisat | fest, spröde |

Im folgenden wird gezeigt, wie sich diese Unterschiede im Elastizitätsverhalten von 2-Komponenten Polyurethanlacken auf Basis dieser Polyisocyanate wiederfinden.

Zu diesem Zweck wurden die vier aufgeführten Isocyanat-Typen mit einem Standard-Polyacrylatharz aus Acrylsäure, Methacrylsäureestern und Styrol, modifiziert mit einem harten Polyester (60 % in Xylol, 3,5 % OH-Gruppen bezogen auf Feststoff) kombiniert und mit Titandioxid pigmentiert (65 % bezogen auf Gesamtbindemittel). Daraus hergestellte Filme wurden 20 Tage bei Raumtemperatur ausgehärtet. Danach wurde die Erichsen-Tiefung in mm bestimmt (Schichtdicke der Filme ca. 40 μm).

| Polyisocyanatkomponente | Erichsen-Tiefung (mm) |
|---|---|
| HDI-Trimerisat | 7-9 |
| Mischtrimerisat | 6-7 |
| Trimerisatgemisch | 0,5-1 |
| IPDI-Trimerisat | 0,5-0,8 |

Aufgrund des stärkeren Weichharzcharakters des Mischtrimerisats zeigt der daraus hergestellte Lackfilm eine für die Praxis ausreichend Elastizität, während das entsprechende Trimerisatgemisch dem Lack die Elastizität nimmt.

**Ansprüche**

1. Verfahren zur Herstellung von Isocyanatgruppen aufweisenden Isocyanuraten durch Trimerisierung eines Teils der Isocyanatgruppen eines aus aliphatischen und cycloaliphatischen Diisocyanaten bestehenden Diisocyanatgemischs in Gegenwart von die Trimerisierung von Isocyanatgruppen beschleunigenden Katalysatoren, Abbruch der Trimerisierungsreaktion, sobald 10-60 % der ursprünglich vorliegenden Isocyanatgruppen trimerisiert sind, wobei der eingesetzte Katalysator durch Erhitzen des Reaktionsgemischs auf eine oberhalb der Zersetzungstemperatur des Katalysators liegende Temperatur und/oder durch Zugabe eines Katalysatorengifts, desaktiviert wird und gegebenenfalls anschließende Entfernung des nicht-umgesetzten Isocyanat-Überschusses durch Dünnschichtdestillation, dadurch gekennzeichnet, daß man ein Gemisch aus Hexamethylendiisocyanat und 1-Isocyanato-3,3,5-trimethyl-5-isocyanatomethyl-cyclohexan im Molverhältnis 4 : 1 bis 1 : 4 einsetzt.

2. Verfahren gemäß Anspruch 1, dadurch gekennzeichnet, daß man die Diisocyanate in einem Molverhältnis zwischen 3 : 1 und 1 : 3 einsetzt.

3. Verfahren gemäß Anspruch 1, dadurch gekennzeichnet, daß man die Diisocyanate in einem Molverhältnis zwischen 2 : 1 und 1 : 2 einsetzt.

4. Verfahren gemäß Anspruch 1 bis 3, dadurch gekennzeichnet, daß man als Katalysator quaternäre Ammoniumhydroxide verwendet.

5. Verfahren gemäß Anspruch 1 bis 4, dadurch gekennzeichnet, daß man als Katalysator quaternäre Ammoniumhydroxide mit mindestens einer, am Stickstoffstoff gebundenen 2-Hydroxyalkylgruppe verwendet.

6. Gemäß Anspruch 1 bis 5 erhaltene, Isocyanatgruppen aufweisende Isocyanurate.

7. Verwendung der gemäß Anspruch 1 bis 5 erhaltenen, Isocyanatgruppen aufweisenden Isocyanurate, gegebenenfalls in mit Blockierungsmitteln für Isocyanatgruppen blockierter Form, als Isocyanatkomponente in Polyurethanlacken.

**Claims**

1. Process for the preparation of isocyanurates containing isocyanate groups by trimerising some of the isocyanate groups of a diisocyanate mixture consisting of aliphatic and cycloaliphatic diisocyanates in the presence of catalysts accelerating the trimerisation of isocyanate groups, stopping the trimerisation reaction as soon as 10-60 % of the isocyanate groups which were originally present are trimerised, the catalyst used being deactivated by heating the reaction mixture to a temperature above the decomposition temperature of the catalyst and/or by adding a catalyst poison, and then optionally removing the non-reacted excess isocyanate by thin-layer distillation, characterised in that a mixture of hexamethylene diisocyanate and 1-isocyanato-3,3,5-trimethyl-5-isocyanato methyl-cyclohexane is used in a mole ratio of 4 : 1 to 1 : 4.

2. Process according to Claim 1, characterised in that the diisocyanates are used in a mole ratio between 3 : 1 and 1 : 3.

3. Process according to Claim 1, characterised in that the diisocyanates are used in a mole ratio between 2 : 1 and 1 : 2.

4. Process according to Claim 1 to 3, characterised in that quaternary ammonium hydroxides are used as the catalyst.

5. Process according to Claim 1 to 4, characterised in that quaternary ammonium hydroxides having at least one 2-hydroxy-alkyl group bound to nitrogen are used as the catalyst.

6. Isocyanurates containing isocyanate groups and obtained according to Claim 1 to 5.

7. Use of the isocyanurates containing isocyanate groups and obtained according to Claim 1 to 5, optionally in a form blocked with blocking agents for isocyanate groups, as isocyanate component in polyurethane lacquers.

**Revendications**

1. Procédé de préparation d'isocyanurates portant des groupes isocyanate par trimérisation d'une partie des groupes isocyanate d'un mélange de diisocyanates consistant en diisocyanates aliphatiques et cycloaliphatiques en présence de catalyseurs accélérant la trimérisation des groupes isocyanate, interruption de la réaction de trimérisation dès que 10 à 60 % des groupes isocyanate présents à l'origine sont trimérisés, le catalyseur mis en œuvre étant désactivé par chauffage du mélange de réaction à une température supérieure à la température de décomposition du catalyseur et/ou par addition d'un poison de catalyseur et, le cas échéant, élimination subséquente de l'excès d'isocyanates non converti par distillation en couche mince, caractérisé en ce que l'on met en œuvre un mélange d'hexaméthylène-diisocyanate et de 1-isocyanato-3,3,5-triméthyl-5-isocyanatométhyl-cyclohexane dans un rapport molaire de 4 : 1 à 1 : 4.

2. Procédé selon la revendication 1, caractérisé en ce que l'on met en œuvre les diisocyanates à un rapport molaire de 3 : 1 à 1 : 3.

3. Procédé selon la revendication 1, caractérisé en ce que l'on met en œuvre les diisocyanates à un rapport molaire de 2 : 1 à 1 : 2.

4. Procédé selon les revendications 1 à 3, caractérisé en ce que l'on utilise en tant que catalyseurs des hydroxydes d'ammonium quaternaire.

5. Procédé selon les revendications 1 à 4, caractérisé en ce que l'on utilise en tant que catalyseurs des hydroxydes d'ammonium quaternaire portant au moins un groupe 2-hydroxyalkyle fixé sur l'azote.

6. Isocyanurates portant des groupes isocyanate obtenus par un procédé selon les revendications 1 à 5.

7. Utilisation des isocyanurates portant des groupes isocyanate obtenus par un procédé selon les revendications 1 à 5, éventuellement sous forme bloquée par des agents bloquants des groupes isocyanate, en tant que composant isocyanate dans des vernis de polyuréthanne.